(19) Europäisches Patentamt
European Patent Office
Office européen des brevets

(11) Publication number: **0 174 289**
**A1**

(12) # EUROPEAN PATENT APPLICATION

(21) Application number: 85850277.6

(22) Date of filing: 05.09.85

(51) Int. Cl.[4]: C 12 Q 1/32
G 01 N 33/52, G 01 N 21/64
A 01 C 1/02

(30) Priority: 06.09.84 DK 4277/84

(43) Date of publication of application:
12.03.86 Bulletin 86/11

(84) Designated Contracting States:
DE FR GB IT NL

(71) Applicant: DE FORENEDE BRYGGERIER A/S
Vesterfaelledvej 100
DK-1799 Copenhagen V(DK)

(72) Inventor: Aage, Jensen Svend
Selma Lagerloefs alle 15
DK-2860 Soeborg(DK)

(72) Inventor: Mollwitz, Heltved Flemming Sdr. Fasanvej 94B
1st Floor Apartment No. 35
DK-2500 Valby(DK)

(74) Representative: Ström, Tore et al,
Ström & Gulliksson AB Studentgatan 1 P.O. Box 4188
S-203 13 Malmö(SE)

(54) **Method and apparatus for the determination of the vitality in seeds.**

(57) The vitality in seeds, which contain one or more dehydrogenases, is determined by producing a section in the seed's embryo, activating one or more dehydrogenases with one or more protondonor(s) and one or more protonacceptor(s) by the application of these to the section in the embryo, whereupon the embryo's section is made fluorescent by applying a precursor to a fluorescent dyestuff, which is developed by the addition of a catalyst. The remaining section of the seed is counter-dyed in a known manner, and the seed is radiated with light at a wavelength which activates the fluorescent dyestuff, while at the same time the emitted fluorescence is measured at another wavelength, in that prior to the measurement of the fluorescence, it is possible to effect a scanning by which reflection from the dyed embryo is measured.

An apparatus for use in executing the method comprises a light source (1) for the transmission of light for activation of the fluorescent dyestuff, a receiver (2) for simultaneous observation or measurement of the emitted fluorescence, a scanning table (3) for use in the investigation of many seeds placed in matrix form, a control unit (5) for the movement of the scanning table, means for the transfer of the results to a memory and/or printout unit (4), and preferably a diaphragm and focusing arrangement (7) whereby all the seeds can be measured singly or collectively.

EP 0 174 289 A1

Croydon Printing Company Ltd

# METHOD AND APPARATUS FOR THE DETERMINATION OF THE VITALITY IN SEEDS.

The present invention relates to a method and apparatus for the determination of the vitality in seeds which contain one or more dehydrogenases.

The determination of the vitality of seeds, i.e. the probability that the seed can be expected to germinate and grow into a plant with a healthy yield, is of very great importance, for example in the evaluation by brewers of the quality of barley to be used as malt, and also to seed dealers and suppliers in the checking of seeds, for example for field crops such as grass, and including cereal crops, e.g. wheat, rye, barley, oats, durra or maize, seed crops e.g. rape, mustard, beans or peas or other crops such as beet, radishes, tomatoes or carrots.

With vitality investigations, a quick answer is obtained if the investigation is carried out with ungerminated seeds, so that it is not necessary to wait for the germination and thereafter evaluate the germination percentage and the health of the resulting sprouting.

From US patent no. 2,921,598, an apparatus is known for the determination of the vitality of seeds, this being effected via a time-consuming colouring which provides a contrast between healthy and unhealthy embryos in split seeds, after which a subjective evaluation of the coloured embryos must be made. By this method there is a relatively large part of the seeds which can be difficult to classify.

In SU patent no. 510,186, there is described a vitality measurement by UV-radiation and measurement of the luminescence of the vapours given off by the seeds after these have been treated chemically with $NO_2$ and $I_2$, but this method is lengthy and inconvenient to carry out.

In general it can be maintained that the colouring of embryos according to US patent no. 2,921,598 and measuring of the colour intensity after producing an incision in the seed, is slow, in that the processes take a time in the region of ½-1 hour, even though heating is carried out to accelerate the dyeing reactions, while at the same time the results achieved are not clear.

The object of the present invention is to provide a method for the determination of the vitality in seeds, said method being quick to carry out and giving a result which does not need to be evaluated subjectively by a person, but is easy to classify.

It has now proved that it is possible to carry out a vitality measurement of seeds by measuring the dehydrogenase activity in the embryo, in that it has been ascertained that if there are active enzymes in the embryo, the seed will be capable of germinating or living. The possibility is thus achieved for a quick, simple and reliable vitality determination which, to a very wide degree, can be automated.

The invention is based on the fact that active enzymes (dehydrogenases) in the embryo can be activated in situ by means of a protondonor and a protonacceptor and give rise to the conversion of a precursor of a fluorescent dye to the dyestuff itself, which colours the embryo. After counter-dyeing of the rest of the seed, one can then by fluorescence measurement obtain a quick and reliable determination of whether there are active enzymes present, and this method is considerably more selective and many times (approx. 1000 times) more sensitive than the method known from US patent no. 2,921,598. At the same time, there are very few interference factors and the evaluation is more reliable, the reason being that the result is reinforced when there is an activity, so that a clear graduation becomes apparent between existing enzyme activity and no activity. Moreover, dyeing and observation or measurement by the method according to the invention can be carried out in a few minutes.

The method according to the invention is characteristic in that,

a) a section is produced in the seed's embryo,

b) one or more dehydrogenases are activated with one or more protondonor(s) and one or more protonacceptor(s) by applying these to the section in the embryo,

c) the section in the embryo is made fluorescent by the application of a precursor of a fluorescent dye of the type which reacts with the protonised protonacceptor and is thereby developed by the addition of a catalyst,

d) the seed's remaining section is counter-dyed in a manner known per se, and

e) the seed is radiated with light at a wavelength which activates the fluorescent dyestuff, and the resulting fluorescence emitted is observed or measured at another wavelength.

The method will be able to be executed, for example with the use of a dehydrogenase such as ethanol dehydrogenase, lactic acid dehydrogenase, glutaric acid dehydrogenase, succinic acid dehydrogenase or glucose-6-phosphate dehydrogenase or another dehydrogenase in the seed, which will therefore be able to be determined by the method. The protondonor which is used is expediently ethanol, lactic acid, glutaric acid, succinic acid and glucose-6-phosphate respectively.

As protonacceptor is used, for example, $NAD^+$ (nicotinamide-adenine dinucleotide) or $NADP^+$ (nicotinamide-adenine dinucleotide phosphate).

As fluorescent dyestuff it is preferable to use resazurin which, by the reactions, is converted to resorufin which fluoresces in the visible range, whereby the observations or the measurements can be carried out very simply by visual evaluation, e.g. with a suitable microscope or by means of a photodetector.

To promote the conversion of resazurin to resorufin a catalyst is added, and for this can be used, for

example, diaphorase or phenazinmethosulphate.

The counter-dyeing of the seed's remaining section, which mainly contains starch, is expediently effected with an iodine solution or a Coomassie Brilliant Blue R 250 (Color Index No. 42660) or G 250 (Color Index No. 42655), whereby a contrast is obtained between embryo and endosperm, thus making it possible to measure the embryo's colour quantitatively and selectively by fluorescence measurement without interference from the endosperm.

By executing the method according to the invention on a number of seeds by the carrying out of observations or photometry by scanning, it is easy to obtain a large number of test results which can be used in calculating the vitality of the seeds.

When the seeds are partially embedded in a plate in an orderly pattern, as presented in claim 4, the sections in the seeds can be produced very simply, and they are arranged so that a subsequent automatic measuring is made possible.

By the measuring of the reflection of light from the dyed section of the seed as presented in claim 5, a measurement of the size or the area of each single embryo is obtained. It is thus possible to compare the area of the embryo with the measured fluorescence, hereby allowing compensation to be made for a lower fluorescence from a seed which has a small section in the embryo, and thus a greater reliability is achieved in carrying out the vitality determination, the reason being that the graduation between the existing and the lacking activity becomes more clear. The reflection measurement can be carried out before or after the fluorescence measurement.

Such a fixing of that area on which the measurement is to be carried out can also be effected by first scanning the dyed and counter-dyed seed, while the reflected light is measured, after which an average effective size of section is calculated, followed by a measurement of the total fluorescence for all the seeds in a sample, for example 100 seeds.

The light which is used for such reflection measurement shall be such that it is reflected by the dyed embryo but not by the counter-dyed rest of the seed section. An expedient illumination for reflection measurement will be visible light, e.g. green, in that the endosperm is dyed "black" by both the iodine solution as well as Coomassie Brilliant Blue R250 or G250.

By grinding down to the middle of the seed, as presented in claim 6, it is ensured that no uncertainty or error is introduced for reasons of different orientation and different sections in the seeds being investigated.

When, as presented in claim 7, radiation is carried out at approx. 585 nm, and the emitted fluorescence is observed or measured at approx. 600 nm, a selective measurement is obtained with no interference from the endosperm after counter-dyeing, and it is possible to carry out a simple visual observation

of the fluorescence.

The invention also relates to apparatus for the execution of step e) in claim 1, said apparatus comprising means for the transmission of light at one wavelength, and means for simultaneous observation or measuring of the light which is emitted at another wavelength from the radiated seed.

The apparatus is constructed principally as described in claim 9, with means for carrying out a series of subsequential determinations, and means for transferring the results to a memory or readout unit.

According to the invention, the apparatus can include a diaphragm and focusing arrangement which makes it possible for the measurement on the seeds to be carried out singly or all together.

In the following, the invention will be explained in more detail with reference to the drawing, which illustrates schematically the apparatus according to the invention.

The apparatus, which is generally designated S, comprises a unit 1 for the transmission of light at a desired wavelength which activates the fluorescent dyestuff, e.g. approx. 585 nm, when one wishes to measure fluorescence when using resorufin, and a detector or receiver 2 which detects that light which is emitted from the radiated seed, e.g. at approx. 600 nm, when fluorescence is measured by means of resorufin. This signal is sent to a memory 4 where

it can be stored for later printout together with other results, or to a printout or readout unit where the measured fluorescence is written out or displayed in analogue or digital manner. A digital value is printed out or displayed, preferably automatically, for each seed. The pre-treated seeds, which are cut through and dyed and counter-dyed, are placed in a matrix in a plate which is mounted in a holder or a scanning table 3 capable of being moved in the X and Y direction at variable speed by a control unit 5, so that each seed in turn can be investigated for fluorescence. A total investigation of a sample can thus be effected, and the apparatus can be provided with a calculation unit 6 which determines the vitality. If desired, a diaphragm and focusing arrangement 7 can be introduced in the light path in order to control the illumination and ensure that only one seed at a time is measured, or the diaphragm and focusing arrangement can be set for the measurement of all seeds at the same time. The individual component parts (light source, photometer, scanning table) are designed in accordance with the general knowledge of those familiar with the art, so that they fulfil the object of the present invention.

When it is desired to scan the sectioned seeds in order to determine the sections' area, it can be expedient for the apparatus to be equipped with a filter system which allows reflection to be measured when using visible light, for example green.

In a simple form, the apparatus can be executed in such a way that it comprises a unit for the transmission of light at a wavelength which activates the fluorescent dyestuff, e.g. 585 nm, a scanning table which can be moved in the X and Y direction either manually or by means of a control unit, a diaphragm and focusing arrangement for controlling the illumination, and means for the visual observation of fluorescence from the exposed seed, this execution also being an aspect of the present invention, whereby a very simple and inexpensive apparatus for the determination of the vitality of seeds is achieved.

The method according to the invention is explained in more detail by means of the following example:

EXAMPLE

Dyeing of seeds:

The reactions are elucidated with ethanol dehydrogenase as example. The following reactions occur:

(1) $CH_3CH_2OH + NAD^+ \xrightarrow{\text{alcohol dehydrogenase}} CH_3CHO + NADH + H^+$

(2) $NADH + H^+ + \text{resazurin} \xrightarrow{\text{diaphorase}} NAD^+ + \text{resorufin} + H_2O$

Procedure:

Seeds for testing or investigation are selected following a general system of sample selection. The seeds are arranged in a matrix by means of a hole-plate, where the seeds are oriented and fall down

into a mould with depressions corresponding to the seeds. The seeds are fixed in a plate of a plastic material, for example wax, plasticine or clay, and ground down to the plate, thus producing a section through the embryo in the individual seeds. The halved seeds are dyed in the following manner:

A $10^{-3}$M NAD solution in 50% ($^{v}/v$) ethanol and 50% ($^{v}/v$) 0.1M tris buffer (pH-value 9.0) is applied in drop form. Immediately afterwards, the halved seeds are given a $2 \cdot 10^{-4}$ M resazurin solution in 2-methoxy ethanol followed by a solution of 8.0 U/ml diaphorase in 0.1 M tris buffer (pH-value 9.0).

The reactions (1) and (2) occur thereafter according to the above, and are concluded after a couple of minutes.

The halved seeds are dried off and the endosperm is counter-dyed with a solution of 5g KI and 0.5g $I_2$ in 200 ml water. The iodine will hereby selectively bind itself to the starch, which is found exclusively in the endosperm in a manner known per se.

Instead of diaphorase, phenazinmethosulphate can be used as catalyst, and instead of dyeing with $KI/I_2$, the starch can be dyed by means of Coomassie Brillian Blue R 250 or G 250. In the reactions (1) and (2), NADP can be used instead of NAD, similarly in 50% ethanol/50% tris buffer. If desired, all reagents with respect to the reactions (1) and (2) can be applied at the same time.

Measurement:

The dyed seeds in the plate are transferred to a spectrofluorometer where they are exposed to light with a wavelength of approx. 585 nm (+/- 25 nm, depending on the pH-value), and at the same time measured at a wavelength of approx. 600 nm (+/- 25 nm, depending on the pH-value). Each seed in position is measured, and the measured value is automatically written out digitally, after which the control unit brings the next seed into position. The results can either be handled mechanically or by evaluation by the person carrying out the measurements, for the calculation of the percentage of seeds which give fluorescence and are thereby alive. Alternatively, all the seeds can be measured at the same time, whereby an average measurement is obtained. On the basis of experience gained from cultivation research, with the measuring results achieved from the above one can quickly arrive at a very reliable estimation of the germination capability etc. of a selected seed sample, whereby in a short time it is possible to evaluate whether a delivery of seed fulfils the norms which are required to be fulfilled for the use contemplated.

C L A I M S

1. Method for the determination of the vitality in seeds which contain one or more dehydrogenases, c h a r a c t e r i z e d in that

a) a section is produced in the seed's embryo,

b) one or more dehydrogenases are activated by one or more protondonor(s) and one or more protonacceptor(s) by the application of these to the seed's section,

c) the section of the seed is made fluorescent by the application of a precursor to a fluorescent dyestuff of the type which reacts with the protonised protonacceptor, and is thereby developed by the addition of a catalyst,

d) the remainder of the seed's section is counterdyed in a manner known per se, and

e) the seed is radiated with light at a wavelength which activates the fluorescent dyestuff, and the resulting emitted fluorescence is observed or measured at another wavelength.

2. Method according to claim 1, c h a r a c t e r i z e d in that resazurin is used as precursor to a fluorescent dyestuff, diaphorase or phenazinmethosulphate is used as catalyst, and an iodine solution or Coomassie Brilliant Blue R 250 (Color Index No. 42660) or G 250 (Color Index No. 42655) is used as the counter-dyeing agent.

3. Method according to claims 1 or 2, c h a r a c t e r i z e d in that it is executed on a number of seeds singly or collectively, in that the individual measurements are carried out by X-Y scanning.

4. Method according to claim 3, c h a r a c t e r i z e d in that the seeds are partially fixed in an orderly pattern, e.g. a matrix pattern, in a plate, and the sections are produced by grinding down.

5. Method according to any of the claims 1-4, c h a r a c t e r i z e d in that scanning is effected with visible light which is reflected by the dyed embryo, further to which the measured reflection is used for correction of the measured fluorescence.

6. Method according to claim 4, c h a r a c t e r i z e d in that the seeds are half-molded down in the plate, and that grinding down to the middle of seed is carried out.

7. Method according to any of the claims 1-6, c h a r a c t e r i z e d in that the seed or the seeds are radiated with light at approx. 585 nm, and that measurement of the emitted fluorescence is carried out at approx. 600 nm.

8. Apparatus for the execution of the method according to claim 1, c h a r a c t e r i z e d in that it comprises means for the transmission of light at one wavelength, and other means for simultaneous observation or measurement of fluorescence at another wavelength.

9. Apparatus according to claim 8, c h a r a c t e r i z e d in that it comprises a scanning table for the investigation of many seeds disposed in matrix form, a control unit for the movement of the scanning table in the X and Y direction, and means for the transfer of the measuring results to a memory or printout unit.

10. Apparatus according to claims 8 or 9, c h a r a c t e r i z e d in that it comprises a diaphragm and focusing arrangement which makes it possible for the measurement on the seeds to be carried out singly or collectively.

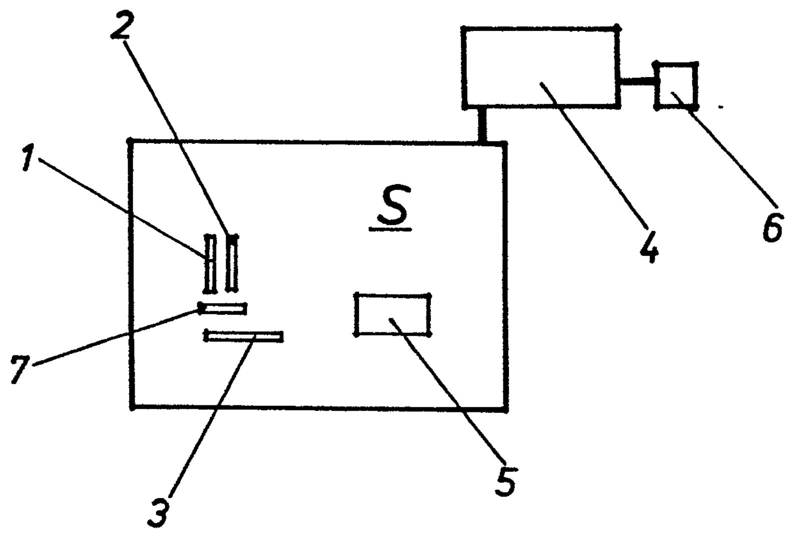

S
1
2
3
4
5
6
7

European Patent Office

# EUROPEAN SEARCH REPORT

0174289 Application number

EP 85850277.6

| Category | Citation of document with indication, where appropriate, of relevant passages | Relevant to claim | CLASSIFICATION OF THE APPLICATION (Int Cl 4) |
|---|---|---|---|
| X | DE - A1 - 3 012 441 (NIPPON KOGAKU K.K.)<br>* Claims 1,5; pages 6-11; especially page 10 *<br>-- | 1,2,7,8 | C 12 Q 1/32<br>G 01 N 33/52<br>G 01 N 21/64<br>A 01 C 1/02 |
| X | US - A - 4 312 945 (YAMADA et al.)<br>* Abstract; column 2, line 41 - column 3, line 42 *<br>-- | 1,7,8 | |
| A | US - A - 4 421 772 (MUNCK et al.)<br>* Abstract *<br>-- | 8 | |
| A | AT - B - 351 173 (KOMMANDITGESELLSCHAFT SCHWARZHAUPT)<br>* Claim 1 *<br>-- | 1,2 | TECHNICAL FIELDS SEARCHED (Int Cl 4) |
| A | AT - B - 310 359 (BOEHRINGER MANNHEIM)<br>* Claim 1 *<br>---- | 1,2 | C 12 Q<br>G 01 N 21/00<br>G 01 N 33/00<br>A 01 C |

The present search report has been drawn up for all claims

| Place of search | Date of completion of the search | Examiner |
|---|---|---|
| VIENNA | 20-11-1985 | SCHNASS |

CATEGORY OF CITED DOCUMENTS

- X particularly relevant if taken alone
- Y particularly relevant if combined with another document of the same category
- A technological background
- O non-written disclosure
- P intermediate document
- T : theory or principle underlying the invention
- E : earlier patent document, but published on, or after the filing date
- D : document cited in the application
- L : document cited for other reasons
- & : member of the same patent family, corresponding document